# EUROPEAN PATENT SPECIFICATION

(11) **EP 3 123 979 B1**
(45) Date of publication and mention of the grant of the patent: **16.09.2020**
(21) Application number: 15769275.7
(22) Date of filing: 23.03.2015
(51) Int. Cl.: A61F 2/16

(54) **INTRAOCULAR LENS**
INTRAOKULARE LINSE
LENTILLE INTRAOCULAIRE

(30) Priority: 28.03.2014 US 201461971867 P; 11.03.2015 US 201514644747
(43) Date of publication of application: 01.02.2017
(73) Proprietor: Mediphacos Indústrias Médicas S/A, CEP:30575-815 - Belo Horizonte - MG (BR)
(72) Inventor: CAMARGOS, Marcelo Duarte, 30220-260 - Belo Horizonte - MG (BR)
(74) Representative: Soldatini, Andrea
(86) International application number: PCT/BR2015/000035
(87) International publication number: WO 2015/143514

(56) References cited:
- EP-A1- 1 502 561
- US-A- 4 808 181
- US-A- 6 129 760
- US-A1- 2003 204 258
- US-A1- 2006 095 127
- US-A1- 2007 168 028
- US-A1- 2012 330 415
- US-B1- 6 942 695

## Description

### Field of the Invention

The present invention belongs to the field of ophthalmologic implants and refers to an intraocular lens provided with haptics with rotation-stabilizing structures, which have been specially developed and intended to avoid rotary displacement of said lens after it is implanted in a patient's eye.

### Background of the Invention

As it is known from the state of the art, intraocular lenses, commonly called IOLs are medical devices used for ocular implant in surgeries for cataract treatment. Generally, lenses are implanted for replacement of the crystalline lens, a natural human eye lens which, in case of suffering from cataract, will become opaque and as a result the passage of light to the retina will not occur, thus compromising the eyesight.

Prescription of an IOL will take into account information about eye refraction such that an IOL is indicated with the best specification for a given patient. Simpler spherical lenses are commonly prescribed to correct distance vision. Hence, after surgery, the patient will need new glasses to have a better near vision. Furthermore, in case the cornea of this patient causes astigmatism in images generated by his eyes, this should also be corrected by using glasses.

There are also lenses called "premium" which add more benefits the patients, such as, for example, multifocal lenses which produce focal points for both near and distance visions, thus meaning that the patient will no longer be dependent on glasses after cataract surgery.

In addition to multifocal lenses, there are the so-called toric lenses which add onto their surfaces a cylindrical component to correct astigmatism. Such functionality may also be added to the multifocal lenses, which will be then known as toric multifocal IOL. It should be pointed out that astigmatism, in this context, occurs when the cornea comprises different radiuses of curvature in different directions. Therefore, a toric IOL needs to have a correction in a well defined and specific direction for a patient having this type of condition.

In the case of toric IOLs, rotation of the lens within the patient's eye along the axis perpendicular to the optic plane represents a problem. This stems from the fact that the toric lens surface as well as the astigmatic cornea has variable curvatures and is characterized by two main axes: the flat axis and the steep axis. For corneal astigmatism compensation, an IOL is implanted in such a way that the steep axis is aligned with the flat axis of the cornea. The correct alignment between these axes ensures a reduction or even the elimination of the effects of astigmatism, wherein a slight misalignment is already sufficient to strongly reduce the IOL efficiency in the astigmatism compensation, thus causing loss of patient's vision quality.

The lens implant direction is determined by specific calculations which take into account all characteristics of patient's cornea. During a surgical procedure, the surgeon implants the lens in the eye and sets its position according to the axis indicated by these calculations. A problem arises when, after surgery, the lens has its original implantation axis changed by the lens rotation around its own optical axis. To correct this problem, the lens must be repositioned, wherein a new surgery is required. After a long period of time, the IOL creates a bond to the capsular bag, and correction is only possible by extracting the lens and implanting a new IOL. Toric IOLs are therefore highly sensitive to rotation.

Generally, deviations produced by the lens rotation are small enough to be tolerated by patients so as to not produce significantly negative effects. Nevertheless, in many cases, such deviation is big enough to reduce the lens efficiency in the astigmatism correction so that realignment of the lens will be required. In the article by "J. S. Till published in "Journal of Cataract and Refractive Surgery", 2002, it is revealed that for 30° deviations, astigmatism correction is null and for 10° deviations the efficiency in the correction drops to 40% compared to the case in which IOL is perfectly aligned. For deviation values below 10°, the effects, in many cases, are acceptable.

IOLs are made of acrylic material and have a central portion responsible for the optical behavior and a peripheral portion at which haptics responsible for fixing the lens to the capsular bag are disposed. Said haptics have the function of keeping the lens always centered and stable to avoid movements in different axes. However, most of the lenses found in the market do not comprise haptics with suitable configurations and with a specific function for stabilizing IOLs when facing rotation. As explained above, it is observed that stabilization is important in surgical procedures to implant toric lenses for correcting astigmatism.

Based on this scenario, one may find available in the market IOLs manufactured by the company *Human Optics,* said IOLs comprising rotation-stabilizing loops. Such lenses are mounted into three pieces, wherein said loops are manually engaged with the optical body of the lenses. Their loops are very thin and long, comprising unidirectional undulations laterally disposed along the own filament, of which the loop consists, which due to its small thickness makes it difficult to position the lens during surgery, which may generate trauma in the eye capsule when the lens is being handled by the doctor.

Thus, and in spite of documents US2003/204258 and US6129760 disclosing surface grooves disposed on at least one of the front and rear faces of the haptic, it is noted that the present state of the art lacks technical efficient solutions to improve and ensure the rotational stability of the intraocular lenses inside the capsular bag, more particularly when such lenses are intended to correct astigmatism.

### Objectives of the Invention

Therefore, in view of the foregoing, one of the objectives of the present invention is to provide an intraocular lens with haptics having a rotation-stabilizing structure capable of effectively avoid the rotation of said lens after its surgical implantation.

More particularly, one of the objectives of the present invention is to provide an intraocular lens whose haptics comprise rotation-stabilizing structures capable of increasing and improving adhesion levels relative to the capsular bag, wherein a better fixation of the lens is achieved.

### Summary of the Invention

The above-mentioned objectives of the present invention are achieved by means of an intraocular lens comprising at least a central body and at least one haptic having rotation-stabilizing structure, where said rotation-stabilizing structure comprises surface grooves disposed on at least one of the upper and lower faces of the haptics, and surface grooves disposed on a lateral surface of said haptic to further increase the adhesion levels relative to the capsular bag, the radius of curvature of said surface grooves having a value ranging from 0.1 mm to 0.5 mm.

Preferably, said surface grooves are concentric, wherein the spacing between them can more preferably assume a minimal value of 0.1 mm and a maximum value of 0.5 mm.

In a particular advantageous embodiment of the present invention, each of the at least one haptic having a rotation-stabilizing structure comprises from one to fifteen surface grooves disposed on at least one of the upper and lower faces of the haptic.

In an alternative embodiment of the present invention, said surface grooves can be arranged along the entire surface of said haptics.

Preferably, such surface grooves are spaced from each other by 0.1 mm to 1.0 mm distance. Further, in a more preferred mode, the radius of curvature of said grooves comprises a value between 0.5 mm and 1.5 mm.

In a particularly advantageous embodiment of the present invention, each of said at least one rotation-stabilizing structure comprises one to fifteen of said surface grooves disposed on the lateral surface of said haptic.

In a preferred embodiment, the intraocular lens, i.e. object of the present invention, comprises a central body and haptics made into one single piece.

Although capable of being applied to any type of intraocular lens, the present invention is particularly advantageous when applied to toric lenses, i.e. those in which the central body has toric format, for astigmatism correction.

### Brief Description of the Drawings

The present invention will be described in detail through the figures below, wherein:
Fig. 1 is a front view of the intraocular lens provided with haptics comprising rotation-stabilizing structures, in accordance with the present invention.
Fig. 2 is a rear view of the intraocular lens provided with haptics comprising rotation-stabilizing structures, in accordance with the present invention.
Fig. 3 is a perspective view of the intraocular lens provided with haptics comprising rotation-stabilizing structures, in accordance with the present invention.

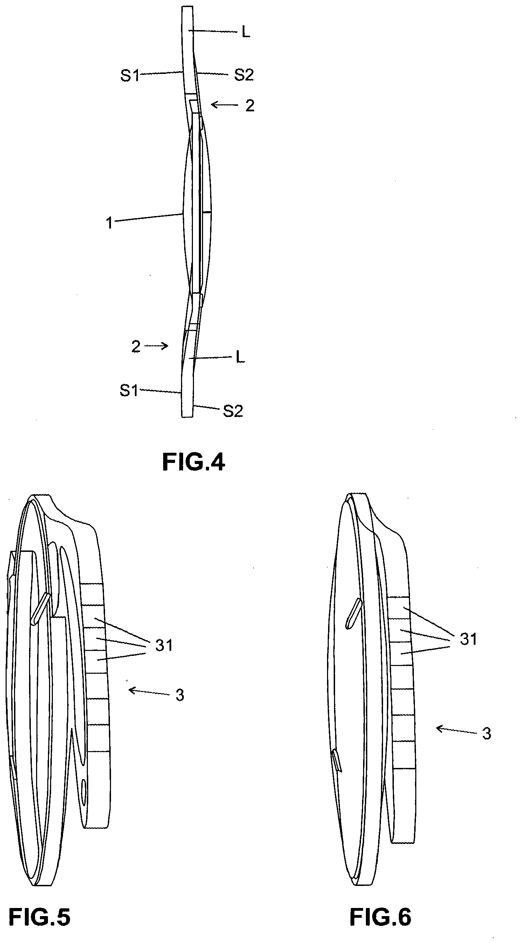

Fig. 4 is a side of view of the intraocular lens provided with haptics comprising rotation-stabilizing structures, in accordance with the present invention.
Fig 5 is an upper view of the intraocular lens provided with haptics comprising rotation-stabilizing structures, in accordance with the present invention.
Fig. 6 is a lower view of the intraocular lens provided with comprising rotation-stabilizing structures, in accordance with the present invention.
Fig. 7 is an amplified perspective view of the haptic comprising rotation-stabilizing structures, in accordance with the present invention.

### Detailed Description of the Invention

In accordance with the schematic figures mentioned above, some examples of the possible embodiments of the present invention will be described in more details below but a in merely exemplificative and not limitative manner, since the object of the present invention can comprise different details and structural and dimensional aspects without however departing from the desired scope of protection.

Hence, the present invention refers to an intraocular lens comprising at least a central body 1 and at least one haptic 2 provided with a rotation-stabilizing structure 3, wherein said rotation-stabilizing structure 3 comprises surface grooves 31 disposed on at least one of the front S1 and rear S2 faces of the haptic 2, specifically responsible of adhesion increase of the intraocular lens towards the surface of the capsular bag.

Preferably, said surface grooves 31 are segments of concentric circles, wherein the spacing between same can also preferably assume a minimum value of 0.1 mm to a maximum value of 0.5 mm.

The radius of curvature of said surface grooves 31 has a value ranging from 0.1 mm to 0.5 mm.

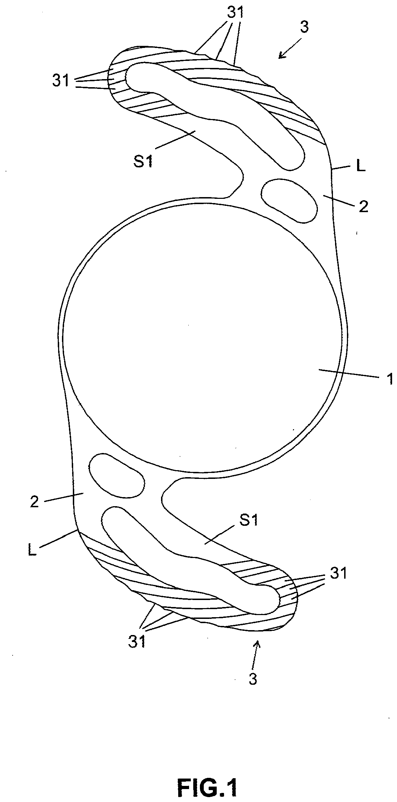

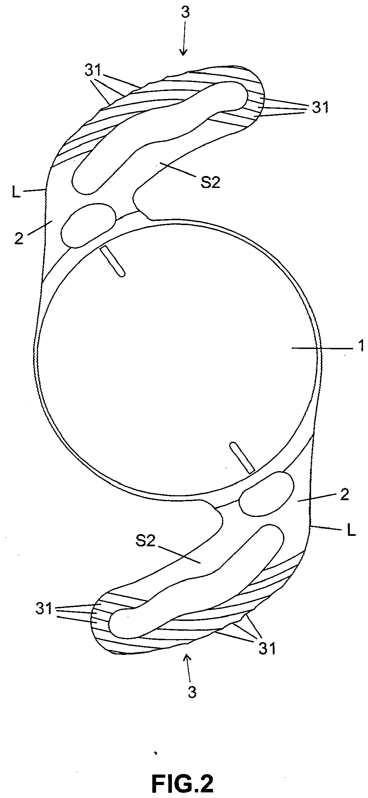

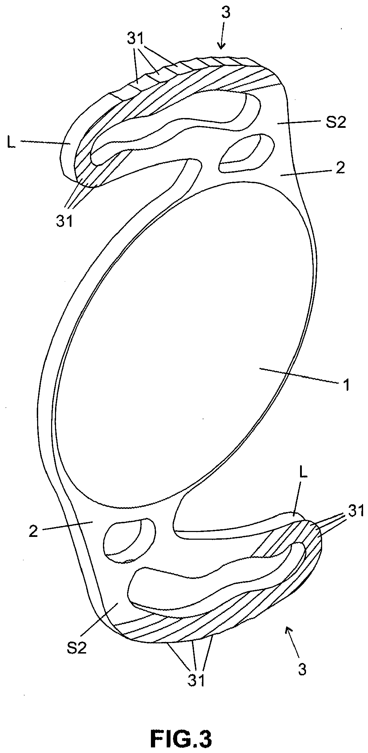

In a preferably advantageous embodiment of the present invention, each of the at least one haptic comprising a rotation-stabilizing structure 3 comprise one to fifteen surface grooves 31. In case said haptic 2 having a rotation-stabilizing structure 31 is provided with central openings, for example, some of its surface grooves 31 can be interrupted in two different portions, as shown in Figs. 1, 2, and 3.

Further, such grooves can be disposed such that they will occupy the entire surface of front S1 and rear S2 faces of said haptic 2 to ensure more safety against undesired rotation of the intraocular lens.

In order to obtain more adhesion relative to the capsular bag, the presently disclosed intraocular lens further comprises surface grooves 31 also distributed on the lateral surface L of its at least one haptic 2. Such surface grooves 31 are spaced from each other, preferably by a 0.1 mm to 1.0 mm distance, and in a more advantageous form, they have a radius of curvature ranging between 0.5 mm and 1.5 mm.

Optionally and in accordance with an advantageous embodiment of the present invention, each of said at least one haptic 2 having a rotation-stabilizing structure 3 comprises one to fifteen surface grooves 31 disposed on its lateral surface L, thus ensuring an increase in the levels of adhesion of the lens to the capsular bag.

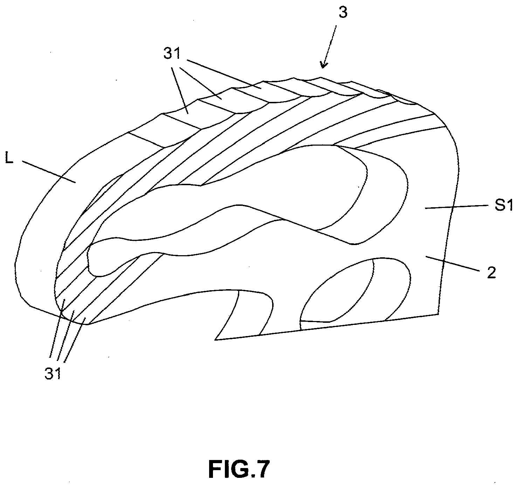

In this sense, Fig. 7 illustrates in detail an exemplificative and particularly advantageous embodiment of the present invention, in which the haptic 2 has a rotation-stabilizing structure 3, wherein six surface grooves 31 are disposed on the front S1 and rear S2 faces of the haptic 2, with a spacing of 0.2 mm and a radius of curvature of 0.35 mm, in addition to eight surface grooves 31 disposed on its lateral surface L, with a spacing of 0,4 mm and a radius of curvature of 0.8 mm.

Although capable of being applied to any type of intraocular lens, the present invention is particularly advantageous when applied to toric lenses, which are those having the central body presenting toric format, for astigmatism correction.

As already formerly said, this is due to the fact that astigmatism occurs when the face of a refractive element contains different radiuses of curvature in different directions. Because of that, the element comprises a face with toroidal or toric format. By this way, astigmatism correction requires a lens having an also toric format, which is capable of compensating the deviation caused in different directions, which are usually perpendicular between them. To obtain an optimal correction, the relative orientation between the lens and the refractive element (cornea, in the case of human eye) has to be precisely positioned and not be changed over time.

In this situation, the presence of haptics 2 provided with rotation-stabilizing structures 3 is extremely necessary, and the fact that surface grooves 31 are provided in their faces, where there is a contact with the eye capsular bag, ensures that the position of the intraocular lens after surgical intervention is maintained.

Further, taking into account the products available in the market, the presently disclosed intraocular lens produces great advances relative to practicability of the manufacture process and efficiency of the adhesion to the capsular bag. The first advance refers to the fact that the lens can have its central body and its haptics made into one single piece, different from what is disclosed in the state of the art. In turn, the second advance occurs because the haptic is not undulated but rather present surface grooves on its front and rear surfaces, and possibly on the side. Said grooves ensure rotational stability without compromising the lens being handled by the doctor or without offering any risk of damage to the patient's eye capsule, which ensure safety to the surgical procedure and higher reliability on the post-operative result, directly implying an enhance in the quality of life of a patient subjected to cataract treatment.

It is important to point out that the description above only intends to describe in an exemplificative manner all the preferred embodiments of the intraocular lens of the present invention. Hence, as understood by a person skilled in the art, the invention contemplates several construction modifications, variations and combinations of the features exerting the same function in substantially the same form to arrive at the same results, which are within the scope of protection limited by the appended claims.

## Claims

1. Intraocular lens comprising at least a central body (1) and at least one haptic (2) comprising a rotation-stabilizing structure (3), said rotation-stabilizing structure (3) comprising surface grooves (31) disposed on at least one of the front (S1) and rear (S2) faces of the haptic (2), **characterized in that** said surface grooves (31) have a radius of curvature between 0.1 mm and 0.5 mm and said rotation-stabilizing structure (3) further comprises surface grooves (31) disposed on the lateral surface (L) of said at least one haptic (2).

2. Intraocular lens, in accordance with claim 1, **characterized in that** said surface grooves (31) are concentric.

3. Intraocular lens, in accordance with claim 1, **characterized in that** said surface grooves (31) are spaced from each other by a 0.1 mm to 0.5 mm distance.

4. Intraocular lens, in accordance with claim 1, **characterized in that** said rotation-stabilizing structure (3) comprises one to fifteen surface grooves (31) disposed on at least one of the front (S1) and rear (S2) faces of said haptic (2).

5. Intraocular lens, in accordance with claim 1, **characterized in that** it comprises surface grooves (31) disposed along the entire surface of at least one of said front (S1) and rear (S2) faces of said haptic (2).

6. Intraocular lens, in accordance with claim 1, **characterized in that** said surface grooves (31) disposed on the lateral surface (L) of said at least one haptic (2) are spaced from each other by a 0.1 mm to 1.0 mm distance.

7. Intraocular lens, in accordance with claim 6, **characterized in that** said surface grooves (31) disposed on the lateral surface (L) of said at least one haptic (2) have a radius of curvature between 0.5 mm and 1.5 mm.

8. Intraocular lens, in accordance with claim 6, **characterized in that** it comprises one to fifteen surfaces (31) disposed on the lateral surface (L) of said at least one haptic (2).

## Patentansprüche

1. Intraokulare Linse, enthaltend wenigstens einen zentralen Körper (1) und wenigstens eine Haptik (2), die eine eine Drehung stabilisierende Struktur (3) enthält, wobei die eine Drehung stabilisierende Struktur (3) Oberflächennute (31) enthält, die an wenigstens einer der vorderen (S1) und hinteren Seiten (S2) der Haptik (2) angeordnet sind, **dadurch gekennzeichnet, dass** die Oberflächennute (31) einen Radius der Krümmung zwischen 0,1 mm und 0,5 mm hat und die eine Drehung stabilisierende Struktur (3) ferner Oberflächennute (31) enthält, die an der seitlichen Oberfläche (L) der wenigstens einen Haptik (2) angeordnet sind.

2. Intraokulare Linse nach Anspruch 1, **dadurch gekennzeichnet, dass** die Oberflächennute (31) konzentrisch sind.

3. Intraokulare Linse nach Anspruch 1, **dadurch gekennzeichnet, dass** die Oberflächennute (31) voneinander durch einen Abstand von 0,1 mm bis 0,5 mm voneinander beabstandet sind.

4. Intraokulare Linse nach Anspruch 1, **dadurch gekennzeichnet, dass** die eine Drehung stabilisierende Struktur (3) eine bis fünfzehn Oberflächennut(e) (31) enthält, die an wenigstens einer der vorderen (S1) und hinteren Seiten (S2) der Haptik (2) angeordnet sind.

5. Intraokulare Linse nach Anspruch 1, **dadurch gekennzeichnet, dass** sie Oberflächennute (31) enthält, die längs der gesamten Oberfläche von wenigstens einer der vorderen (S1) und hinteren Seiten (S2) der Haptik (2) angeordnet sind.

6. Intraokulare Linse nach Anspruch 1, **dadurch gekennzeichnet, dass** die Oberflächennute (31), die an der seitlichen Oberfläche (L) der wenigstens einen Haptik (2) angeordnet sind, durch einen Abstand von 0,1 mm bis 0,5 mm voneinander beabstandet sind.

7. Intraokulare Linse nach Anspruch 6, **dadurch gekennzeichnet, dass** die Oberflächennute (31), die an der seitlichen Oberfläche (L) der wenigstens einen Haptik (2) angeordnet sind, einen Radius der Krümmung zwischen 0,5 mm und 1,5 mm haben.

8. Intraokulare Linse nach Anspruch 6, **dadurch gekennzeichnet, dass** sie eine bis fünfzehn Oberflächennut(e) (31) enthält, die an der seitlichen Oberfläche (L) der wenigstens einen Haptik (2) angeordnet sind.

## Revendications

1. Lentille intraoculaire comprenant au moins un corps central (1) et au moins un système haptique (2) comprenant une structure de stabilisation en rotation (3), ladite structure de stabilisation en rotation (3) comprenant des rainures de surface (31) disposées sur au moins l'une des faces avant (S1) et arrière (S2) du système haptique (2), **caractérisée en ce que**
lesdites rainures de surface (31) ont un rayon de courbure compris entre 0,1 mm et 0,5 mm, et que
ladite structure de stabilisation en rotation (3) comprend en outre des rainures de surface (31) disposées sur la surface latérale (L) dudit au moins un système haptique (2).

2. Lentille intraoculaire, selon la revendication 1, **caractérisée en ce que** lesdites rainures de surface (31) sont concentriques.

3. Lentille intraoculaire, selon la revendication 1, **caractérisé en ce que** lesdites rainures de surface (31) sont espacées les unes des autres d'une distance comprise entre 0,1 mm à 0,5 mm.

4. Lentille intraoculaire, selon la revendication 1, **caractérisée en ce que** ladite structure de stabilisation en rotation (3) comprend une à quinze rainures de surface (31) disposées sur au moins l'une des faces avant (S1) et arrière (S2) du dit système haptique (2).

5. Lentille intraoculaire, selon la revendication 1, **caractérisé en ce qu'**ell comprend des rainures de surface (31) disposées le long de toute la surface d'au moins l'une desdites faces avant (S1) et arrière (S2) dudit système haptique (2).

6. Lentille intraoculaire, selon la revendication 1, **caractérisée en ce que** lesdites rainures de surface (31) disposées sur la surface latérale (L) dudit au moins un système haptique (2) sont espacées les unes des autres d'une distance comprise entre 0,1 mm et 1,0 mm.

7. Lentille intraoculaire, selon la revendication 6, **caractérisée en ce que** lesdites rainures de surface (31) disposées sur la surface latérale (L) dudit au moins un système haptique (2) ont un rayon de courbure compris entre 0,5 mm et 1,5 mm.

8. Lentille intraoculaire, selon la revendication 6, **caractérisé en ce qu'**elle comprend une à quinze rainures de surface (31) disposées sur la surface latérale (L) dudit au moins un système haptique (2).
